# EUROPEAN PATENT APPLICATION

(11) **EP 3 239 300 A1**
(43) Date of publication of application: **01.11.2017**
(21) Application number: 15871790.0
(22) Date of filing: 10.11.2015
(51) Int. Cl.: C12P 17/16, C12R 1/465

(54) **METHOD FOR PREPARING STALLIMYCIN**

(30) Priority: 24.12.2014 CN 201410813640
(71) Applicant: Zhejiang Hisun Pharmaceutical Co., Ltd., Taizhou, Zhejiang 318000 (CN)
(72) Inventor: JIANG, Shichun, Taizhou Zhejiang 318000 (CN); JIANG, Huan, Taizhou Zhejiang 318000 (CN)
(74) Representative: Kador & Partner
(86) International application number: PCT/CN2015/094178
(87) International publication number: WO 2016/101720

(57) **Abstract**

Provided is a method for preparing stallimycin. The method comprises: fermenting streptomyces in a fermentation medium comprising a carbon source, a nitrogen source and 3-hydroxy-4-aminobutyric acid, and adding vegetable oil into the fermentation medium during the fermentation.

## Description

### TECHNICAL FIELD

The present invention relates to the field of biopharmacy, especially to a method of preparing stallimycin by fermentation.

### TECHNICAL BACKGROUND

Stallimycin is an antibiotic generated from *Streptomyces netropsis* or *S.distallicus,* the hydrochloride thereof is yellowish white crystalline powder, the molecular formula is C₂₂H₂₇N₉O₄HCL, the molecular weight is 517.975, and the melting point is 184∼187°C. Stallimycin is soluble in water, but it is not soluble in organic solvents. The product should be stored in conditions away from light and moisture. Stallimycin has inhibiting effect for Gram-positive bacteria, fungus and virus, the mechanism of action thereof is mainly to inhibit the synthesis of DNA. Stallimycin is mainly used for the skin and mucosal infection caused by herpes simplex, herpes zoster and vaccinia virus etc. It is also an oligopeptide antibiotic with certain anticancer activities, it shows an affinity for special sequences of DNA and can recognize DNA, based on its control for expression of small molecule targeting sequence and the gene switch, it can be used as anti-malarial and anti-angiogenesis drugs. Stallimycin is a kind of antibiotics with lower toxity and higher effectiveness for clinical application, and there are few researches on stallimycin in China, the product is mainly imported. Some references such as Arcamone, F; Penco, S; Orezzi, P.G; Nicolella, V ;Pirelli ,A.Nature1964,203,1064; Michaelw.Van Dyke,Robert P.Hertzberg Peter B. Dervian Proc.Natl.Acad.sci.USA1982,79:5470-5474; Stockert JC,Castillo P D Bella JL.DNA-induced distamycin A fluorescence. Histochemistry,1990.94:45-47 ; Baliga R, Crothers DM.The Kinetic basis for sequence discrimination by distamycin A Jam Chem soc 2000.122:11751-11752 have described some contents such as the physicochemical properties and the antitumor activities of stallimycin and its analogues. The invention patent of the Italian company Pharmacia (publication number CN1468099A) has described the antitumor efficacy of various stallimycin derivatives, which widened its application prospect. Hori et al studied the property of inducing fragile site of stallimycin, which provides a clue for the instability of chromosomes of human beings caused by stress factors. Chinese patent (publication number CN86104774A) describes the preparation method of stallimycin derivatives. However, the advanced fermentation methods for preparing stallimycin are not reported in domestic and foreign literatures. The low level of fermentation, incomplete process and insufficient expression of the good properties of strains of the process for producing stallimycin at present result in some disadvantages of stallimycin such as low titer, large production fluctuation, low yield and high cost etc. In the Selection of High Distamycin Producing Strain, Jiang Shichun etc. (Acta Laser Biology Sinica, 2014, 23(2), 189-192), a mutant strain *Streptomyces distallicus* DZ206 is obtained by mutation, the yield of the mutant strain is 1.38 fold of that of the starting strain *Streptomyces distallicus* D32, providing a way to increase the production level of stallimycin. However, as the fermentation process is not further optimized, the potential of the *Streptomyces distallicus* DZ206 strain cannot be fully excavated, there's limitation to large increase of the yield. Therefore, the work of searching for a high efficient method for preparing stallimycin is continuing.

### SUMMARY OF THE INVENTION

The present invention also provides a high efficient method for preparing stallimycin, comprising the step of fermenting streptomyces that can produce stallimycin in a fermentation medium comprising an available carbon source, an available nitrogen source and 3-hydroxy-4-aminobutyric acid, and the step of adding vegetable oil into the fermentation medium during the fermentation.

In a preferred embodiment, the streptomyces that can produce stallimycin is selected from *Streptomyces distallicus* NRRL NO.2886, *Streptomyces distallicus* D32, *Streptomyces distallicus* DZ206.

In a preferred embodiment, the weight concentration of the 3-hydroxy-4-aminobutyric acid (the structure is shown below, and it is called HABA for short) in the fermentation medium is 0.005%-0.05%, preferably 0.01%.

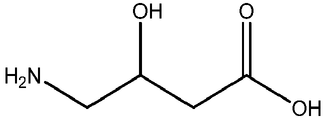

In a preferred embodiment, wherein the fermentation comprises the step of adding vegetable oil into the fermentation medium preferably at the time when fermentation has been conducted for 48-120 hours.

In a preferred embodiment, the interval between the additions of vegetable oil is preferably once every 24 hours.

In a preferred embodiment, the weight ratio of the amount of vegetable oil added for each time to the fermentation medium is preferably 0.3-1.0%, more preferably 0.5%; the weight ratio of the total amount of the added vegetable oil to the fermentation medium is preferably 1-4%, more preferably 2%.

In a preferred embodiment, the vegetable oil is preferably selected from rapeseed oil, sunflower oil, peanut oil, soybean oil, olive oil or the mixtures thereof, more preferably, soybean oil.

In a preferred embodiment, the weight concentration of the available carbon source in the fermentation medium is preferably 4-10%, more preferably 5-8%, most preferably 6%; the weight concentration of the available nitrogen source in the fermentation medium is preferably 2-7%, more preferably 3-6%, most preferably 5%.

In a preferred embodiment, the available carbon source is preferably selected from lactose, maltose, dextrin, cassava flour, corn starch, glucose, wheat starch, potato starch, glycerol, vegetable oil or the mixtures thereof, more preferably glucose, dextrin, soybean oil or the mixtures thereof; the available nitrogen source is preferably selected from soybean meal, yellow soybean cake meal, yeast powder, corn steep liquor, dried corn steep liquor powder, fish meal, peptone, peanut cake powder, cottonseed cake meal, bran, gluten powder, ammonium sulfate, ammonium phosphate, ammonium chloride, ammonium nitrate or the mixtures thereof, more preferably soybean meal, gluten powder or the mixture thereof.

In a preferred embodiment, the temperature of fermentation is preferably 20-40°C, the time of fermentation is preferably 144-192 hours, the pH of the fermentation medium is 6.0-9.0.

In the present invention, *Streptomyces distallicus* NRRL NO.2886 can be seen in patent US3190801; *Streptomyces distallicus* D32 and *Streptomyces distallicus* DZ206 can be seen in the article: Selection of High Distamycin Producing Strain, Jiang Shichun etc. (Acta Laser Biology Sinica, 2014, 23(2), 189-192).

The spore slant culture medium mentioned in the present invention comprises (1) 3-5% available carbon source, suitable available carbon source comprises corn starch, cassava flour, dextrin, lactose, glucose, maltose and the like; (2)1-2% available nitrogen source, suitable available nitrogen source comprises yeast powder, yeast extract, casein, peptone, corn steep liquor, fish meal, ammonium sulfate, ammonium phosphate and the like.

The preparation of the spore slant culture medium, the preparation of the seed culture medium and the fermentation culture process according to the present invention are as follows:

### Preparation of spore slant culture medium:

The prepared spore slant culture medium is subjected to moist heat sterilization at a temperature of 121 to 125°C and a pressure of 0.10 to 0.13 MPa for 30 minutes, a solid medium is preferred, the suitable culture temperature is 20 to 40°C, the optimum temperature is 29 ± 2°C, the incubation time is 4-14 days, preferably 6-12 days.

### Preparation of seed culture medium:

The seed culture medium of the present invention contains (1) 1-4% available carbon source, suitable available carbon source comprises starch, dextrin, glycerol, glucose, lactose, maltose and the like; 1-4% available nitrogen source, suitable available nitrogen source comprise yellow soybean cake meal, yeast powder, peptone, corn steep liquor, bran, gluten powder, ammonium chloride, ammonium nitrate, ammonium sulfate and the like. The medium is subjected to moist heat sterilization at a temperature of 121-125°C and a pressure of 0.10-0.13Mpa for 30 minutes.

After cooling, the slant spore suspension was inoculated into the seed culture medium to be cultivated, the loading quantity of the shake flask is 10-15% of the volume of the flask, the medium is filtered by 8 layers of gauze, the rotation speed of the shaker is 200-240rpm, the loading quantity of the seed tank is 65-75%, the ventilation ratio is 1:1v/v/m, the stirring speed is 200-240rpm, the suitable culture temperature is 20-40°C, the optimum temperature is 29 ± 2°C, the culture time is 20-48 hours, and the mycelial concentration is 5-20%.

### Fermentation culture

The fermentation medium of the present invention contains: (1) 4-10% available carbon source, suitable available carbon source comprises corn starch, dextrin, glycerol, glucose, cassava powder, potato powder, wheat starch, lactose, maltose, vegetable oil and the like, preferably dextrin, glucose, soybean oil or the mixtures thereof; (2) 2-7% available nitrogen source, suitable available nitrogen source comprises yellow soybean cake meal, peanut cake powder, cotton seed cake meal, soybean meal, yeast powder, peptone, corn steep liquor, dried corn steep liquor powder, fish meal, bran, gluten powder, ammonium chloride, ammonium nitrate, ammonium sulfate, ammonium phosphate and the like, wherein soybean powder, gluten powder or the mixture thereof are preferred. During the fermentation (48-120 hours), 1-4% of available vegetable oil selected from rapeseed oil, sunflower oil, peanut oil, soybean oil, olive oil or the mixtures thereof is added, preferably soybean oil, rapeseed oil, more preferably soybean oil, preferably at a concentration of 1 to 4% and the optimum concentration is 2%. The concentration of the available HABA is 0.005-0.05%, preferably 0.01%. The culture medium is subjected to sterilization at 121-125°C and 0.10-0.13Mpa for 30 minutes. After cooling, the slant spore suspension is inoculated into the seed culture medium for cultivation, the loading quantity of the shake flask is 10-12% of the volume of the flask, the medium is filtered by 8 layers of gauze, the rotation speed of the shaker is 200-240rpm, the loading quantity of fermentation tank is 65-75%, the ventilation ratio is 1:0.8 v/v/m, the stirring speed is 200-240rpm, the culture temperature is 20-40°C, the optimum temperature is 29 ± 2°C, the culture time is 144-192 hours, and the mycelia concentration is 30-50%.

### Determination of the titer of stallimycin

High performance liquid chromatography (HPLC) method for the detection of stallimycin:
After fermentation, 3 fold (volume) of 80% ethanol is added to the fermented mycelia liquid, the mixture is soaked for 15-30 minutes, filtered, the filtrate is taken for HPLC detection, the detection conditions are: column: Shandon Hypersil 250*2.1mm, column temperature: 40 °C, mobile phase: solvent A is acetonitrile; solvent B is sodium dodecyl sulfate aqueous solution 12g/L (the preparation method thereof: 12 grams of sodium dodecyl sulfate, 12ml 85% phosphoric acid aqueous solution is added, pure water is added until the volume reaches 1000ml, and then the pH is adjusted to 2.6 with 20% sodium hydroxide aqueous solution). The flow rate of the mobile phase: 0.25ml/min, the wavelength of detection: 304nm. The retention time of stallimycin is 8.2 minutes. The gradient conditions of the mobile phase are as follows:

| Time (minute) | 0 | 2 | 5 | 17 | 19 | 23 | 24 | 25 | 28 |
|---|---|---|---|---|---|---|---|---|---|
| A% | 100 | 35 | 37 | 48.3 | 52 | 56 | 70 | 70 | 35 |
| B% | 0 | 65 | 63 | 51.7 | 48 | 44 | 30 | 30 | 65 |

The present invention has the following advantages over the original process:
The present invention provides a method for efficiently fermenting stallimycin: inoculating streptomyces which can produce stallimycin into a liquid seed culture medium, waiting until the cells are mature, transferring the cells to an optimized fermentation medium and adjusting the process conditions, and by the method of feeding culture medium during the fermentation, the fermentation level is substantially increased, the yield of stallimycin reaches 2.2 fold of the original process, which is at an international leading level. Because of the improvement of the fermentation process, the fermented mycelia has become from the original paste into a granular shape, which is beneficial to the separation of solid and liquid, the extraction yield is thus increased. At the same time, the utilization rate of the device and the raw materials are increased, the cost of production are largely reduced, the method build a foundation for entering into the international market and for localization, thus has significant economic and social benefits:
   (1) By using the method of the invention, the high-yield characteristic of the bacteria can be fully exerted, the mycelia grow vigorously at the early stage of fermentation, the speed of fermentation metabolism is fast, the yield of the antibiotics increases significantly, which is advantageous for decreasing infection rate.
   (2) By using the method of the invention, the viscosity of the material liquid obtained at the end of fermentation is low, the filtration speed is fast, the filtrate obtained is clear, which is advantageous for increasing the extraction yield and the quality of the finished product. The abnormalities such as the productivity of the fermentation in original process is low, the viscosity is high, a paste is formed, the solution cannot be filtered completely, the yield is low and the loss is great have been avoided.
   (3) By using the present invention, HABA is added to the fermentation basal medium, during the culture process, the process of adding vegetable oil is adopted, so that the fermentation level is increased steadily, and the escape of the liquid is avoided at the same time, the tank volume is increased, the fermentation index is increased and the fermentation titer reaches 2.2 fold of the original process, thereby greatly improving the production level, while significantly reducing the cost of production.
   (4)In the invention, HABA is added to the fermentation medium, and the structure and proportion of the carbon and nitrogen nutrient in the medium are adjusted; at the same time, the vegetable oil is added during the culture process, the high yield performance is fully exerted, the antibiotic secretion is quick and the yield is increased, the ratio of the active component is increased, there's less impurities, and the water content of the filtered mycelia residue is low, the filter residue is loose, easy to be loaded and unloaded, the labor intensity is decreased.
   (5) The present invention can be implemented on existing pharmaceutical production equipment without any additional investment.

### EMBODIMENTS

The present invention will be further illustrated by the following examples, to let the present invention be fully understood by those skilled in the art. The examples are provided for illustrating the present invention but not limiting the present invention. Simple modification of the present invention according to the essence of the present invention all belong to the scope protected by the present invention. Unless stated otherwise, the percentages in the present invention are all weight percentages or weight percentage concentrations.

*Streptomyces distallicus* NRRL NO.2886 was disclosed in US 3190801, it can be obtained from Agricultural Research Service Culture Collection in the United States.

*Streptomyces distallicus* D32 and *Streptomyces distallicus* DZ206 were disclosed in "Selection of High Distamycin Producing Strain, Jiang Shichun etc. Acta Laser Biology Sinica, 2014, 23(2), 189-192, it can be obtained from Zhejiang Hisun Pharmaceutical Co., LTD.

### Comparative example 1: Preparation of stallimycin

### (1) Strain: Streptomyces distallicus NRRL NO.2886

### (2) Preparation and culture of slant pore

Formula of the medium (%):glucose 4.5, yeast powder 1.5, sodium chloride 0.3, magnesium sulfate heptahydrate 0.05, calcium carbonate 0.2, ammonium sulfate 0.05, EDTA 0.02, the pH before sterilization was 6.2, it was prepared with nature water, the loading quantity was 50mL in a 250mL eggplant-shaped bottle. The medium was subjected to sterilization at the temperature of 121-125°C and at the pressure of 0.10 to 0.13 MPa for 30 minutes, it was laid aside and became a slant after suitable cooling for later use.

The spore solution of the stallimycin producing strain, *Streptomyces distallicus* NRRL NO.2886 was uniformly coated on the above blank slant, and it was cultivated in an incubator with constant temperature at 29 ± 2 °C for 6-12 days. After the spores were mature, they were ash grey.

### (3) Preparation and culture of shake flask seed culture medium

Formula of the medium (%): glucose 2, corn steep liquor2.5, calcium carbonate 1.0, ammonium sulfate 0.1, potassium dihydrogen phosphate 0.01, pH6.0, it was prepared with drinking water, the loading quantity was 50mL in a 500mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was 7%, and the appearance was pale yellow, it can be observed from a microscope that the mycelia was deeply stained and had obvious branches and segments.

### (4) Preparation and culture of shake flask fermentation medium

Formula of the medium (%):glucose 6, gluten powder 2.5, soybean meal 3, sodium chloride 0.25, calcium carbonate 0.3, potassium dihydrogen phosphate 0.02, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the amount of inoculation was 10%, the culture temperature was 29±2°C, it was cultured on a shaker with a rotation speed of 220 r/min for 168 hours, the concentration of the mycelia in the bottles was about 35%, the pH was about 7.5, the appearance was dark yellow. 3 fold (volume) of 80% ethanol was added to the fermentation medium, soaked for 30 minutes, filtered, the filtrate was collected, and the titer of stallimycin was determined to be 730ug/mL by high performance liquid chromatography (HPLC).

### Comparative example 2: Preparation of stallimycin

### (1) Strain: Streptomyces distallicus D32

### (2) Preparation and culture of slant pore

Formula of the medium (%): glucose 4.5, yeast powder 1.5, sodium chloride 0.3, magnesium sulfate heptahydrate 0.05, calcium carbonate 0.2, ammonium sulfate 0.05, EDTA 0.02, the pH before sterilization was 6.2, it was prepared with nature water, the loading quantity was 50mL in a 250mL eggplant-shaped bottle. The medium was subjected to sterilization at the temperature of 121-125°C and at the pressure of 0.10 to 0.13 MPa for 30 minutes, it was laid aside and became a slant after suitable cooling for later use.

The spore solution of the stallimycin producing strain, *Streptomyces distallicus* D32 was uniformly coated on the above blank slant, and it was cultivated in an incubator with constant temperature at 29 ± 2 °C for 6-12 days. After the spores were mature, they were ash grey.

### (3) Preparation and culture of shake flask seed culture medium

Formula of the medium (%): glucose 2, corn steep liquor2.5, calcium carbonate 1.0, ammonium sulfate 0.1, potassium dihydrogen phosphate 0.01, pH6.0, it was prepared with drinking water, the loading quantity was 50mL in a 500mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was 8%, the appearance was pale yellow, it can be observed from a microscope that the mycelia was deeply stained and they had obvious branches and segments.

### (4) Preparation and culture of shake flask fermentation medium

Formula of the medium (%):glucose 6, gluten powder 2.5, soybean meal 3, sodium chloride 0.25, calcium carbonate 0.3, potassium dihydrogen phosphate 0.02, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the amount of inoculation was 10%, the culture temperature was 29±2°C, it was cultured on a shaker with the rotation speed of 220 r/min for 168 hours, the concentration of the mycelia in the bottles was about 35%, the pH was about 7.9, the appearance was dark yellow. 3 fold (volume) of 80% ethanol was added to the fermentation medium, soaked for 30 minutes, filtered, the filtrate was collected, and the titer of stallimycin was determined to be 1030ug/mL by high performance liquid chromatography (HPLC).

### Comparative example 3: Preparation of stallimycin

### (1) Strain: Streptomyces distallicus DZ206

### (2) Preparation and culture of slant pore

Formula of the medium (%): glucose 4.5, yeast powder 1.5, sodium chloride 0.3, magnesium sulfate heptahydrate 0.05, calcium carbonate 0.2, ammonium sulfate 0.05, EDTA 0.02, the pH before sterilization was 6.2, it was prepared with nature water, the loading quantity was 50mL in a 250mL eggplant-shaped bottle. The medium was subjected to sterilization at the temperature of 121-125 °C and at the pressure of 0.10 to 0.13 MPa for 30 minutes, it was laid aside and became a slant after suitable cooling for later use.

The spore solution of the stallimycin producing strain, *Streptomyces distallicus* DZ206 was uniformly coated on the above blank slant, and it was cultivated in an incubator with constant temperature at 29 ± 2°C for 6-12 days. After the spores were mature, they were ash grey.

### (3) Preparation and culture of shake flask seed culture medium

Formula of the medium (%): glucose 2, corn steep liquor 2.5, calcium carbonate 1.0, ammonium sulfate 0.1, potassium dihydrogen phosphate 0.01, pH6.0, it was prepared with drinking water, the loading quantity was 50mL in a 500mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was 10%, the appearance was pale yellow, it can be observed from a microscope that the mycelia was deeply stained and they had obvious branches and segments.

### (4) Preparation and culture of shake flask fermentation medium

Formula of the medium (%):glucose 6, gluten powder 2.5, soybean meal 3, sodium chloride 0.25, calcium carbonate 0.3, potassium dihydrogen phosphate 0.02, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the amount of inoculation was 10%, the culture temperature was 29±2°C, it was cultured on a shaker with the rotation speed of 220 r/min for 168 hours, the concentration of the mycelia in the bottle was about 35%, the pH was about 7.9, the appearance was dark yellow. 3 fold (volume) of 80% ethanol was added to the fermentation medium, soaked for 30 minutes, filtered, the filtrate was collected, and the titer of Stallimycin was determined to be 2420ug/mL by high performance liquid chromatography (HPLC).

### Comparative example 4: Preparation of stallimycin

### Experiment with small fermentation tank

### (1) The strain and the preparation and culture of slant pore are seen in comparative example 3

### (2) Preparation of shake flask mycelia

Formula of the medium (%): glucose 2, corn steep liquor 2.5, calcium carbonate 1.0, ammonium sulfate 0.1, potassium dihydrogen phosphate 0.01, pH6.0, it was prepared with drinking water, the loading quantity was 200mL in a 1000mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220 r/min for 40 hours, the concentration of mycelia was about 14%, the appearance was pale yellow, it can be observed from a microscope that the mycelia was deeply stained and they had branches and segments.

### (3) Preparation and culture of seed tank

Formula of the medium (%): glucose 2, corn steep liquor 2.5, calcium carbonate 1.0, ammonium sulfate 0.1, potassium dihydrogen phosphate 0.01, pH6.0, it was prepared with drinking water. The loading quantity was 65L in a 100L seed tank, the medium was subjected to sterilization at the temperature of 121-125°C and the pressure of 0.10-0.13Mpa for 30 minutes, after cooling, the mycelia liquid of the shake flask was inoculated, then it was cultured at the temperature of 29±2°C, stirring speed 220 r/min, ventilation ratio 1:1 v/v/m for 40 hours, the concentration of the mycelia was 12%, the appearance was pale yellow, it can be observed from a microscope that the mycelia was deeply stained, thick and strong, and they had obvious branches and segments.

### (4) Preparation and culture of fermentation tank

Formula of the medium (%): glucose 6, gluten powder 2.5, soybean meal 3, calcium carbonate 0.3, potassium dihydrogen phosphate 0.02, soybean oil 0.1, pH6.2, it was prepared with drinking water. The loading quantity was 700L in a 1000L fermentation tank, the medium was subjected to sterilization at the temperature of 121-125°C and the pressure of 0.10-0.13Mpa for 30 minutes, after cooling, the seed liquid was inoculated with the inoculation amount of 10%, the culture temperature was 29±2°C, the stirring rotation speed was 220 r/min, the ventilation ratio was 1:0.8 v/v/m, the fermentation cycle was 168 hours, the concentration of the mycelia was about 40%, the appearance was dark yellow, pH 8.2, after the pretreatment of the fermentation liquid, the titer of stallimycin was determined to be 2470ug/mL by high performance liquid chromatography (HPLC).

### Example 1: Preparation of stallimycin

### (1) Strain: Streptomyces distallicus DZ206

### (2) Preparation and culture of slant spore

Formula of the medium (%): glucose 4.5, yeast powder 1.5, sodium chloride 0.3, magnesium sulfate heptahydrate 0.05, calcium carbonate 0.2, ammonium sulfate 0.05, EDTA 0.02, the pH before sterilization was 6.2, it was prepared with nature water, the loading quantity was 50mL in a 500mL triangular flask. The medium was subjected to sterilization at the temperature of 121-125 °C and at the pressure of 0.10 to 0.13 MPa for 30 minutes, it was laid aside and became a slant after suitable cooling for later use.

The spore solution of the stallimycin producing strain, *Streptomyces distallicus* DZ206 was uniformly coated on the above blank slant, and it was cultivated in an incubator with constant temperature at 29 ± 2°C for 6-12 days. After the spores were mature, they were ash grey.

### (3) Preparation and culture of shake flask seed culture medium

Formula of the medium (%): glucose 2, corn steep liquor 2.5, calcium carbonate 1.0, ammonium sulfate 0.1, potassium dihydrogen phosphate 0.01, pH6.0, it was prepared with drinking water, the loading quantity was 50mL in a 500mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was 12%, the appearance was pale yellow, it can be observed from a microscope that the mycelia was deeply stained and had obvious branches and segments.

### (4) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.005, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the amount of inoculation was 10%, the culture temperature was 29±2°C, after culturing on a shaker with the rotation speed of 220 r/min for 48 hours, 0.5% soybean oil was added every 24 hours (in total, the amount of soybean oil added during the whole fermentation process was about 2%), it was cultivated for 168 hours and put in a bottle. The concentration of the mycelia of the fermentation medium was about 35%, the pH was about 7.8, and the appearance was dark yellow. 3 fold (volume) of 80% ethanol was added to the fermentation medium, soaked for 30 minutes, filtered, the filtrate was collected, and the titer of stallimycin was determined to be 5430ug/mL by high performance liquid chromatography (HPLC).

### Example 2: Preparation of stallimycin

### Experiment with small fermentation tank

### (1) The strain and the preparation and culture of slant spore are seen in example 1

### (2) Preparation of shake flask mycelia

Formula of the medium (%): glucose 2, corn steep liquor 2.5, calcium carbonate 1.0, ammonium sulfate 0.1, potassium dihydrogen phosphate 0.01, pH6.0, it was prepared with drinking water, the loading quantity was 200mL in a 1000mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was about 12%, the appearance was pale yellow, it can be observed from a microscope that the mycelia was deeply stained and had branches and segments.

### (3) Preparation and culture of seed tank

Formula of the medium (%): glucose 2, corn steep liquor 2.5, calcium carbonate 1.0, ammonium sulfate 0.1, potassium dihydrogen phosphate 0.01, pH6.0, it was prepared with drinking water. The loading quantity was 65L in a 100L seed tank, the medium was subjected to sterilization at the temperature of 121-125°C and the pressure of 0.10-0.13Mpa for 30 minutes, after cooling, the mycelia liquid of the shake flask was inoculated, then it was cultivated at the temperature of 29±2°C, stirring rotation speed 220 r/min, ventilation ratio 1:1 v/v/m for 40 hours, the concentration of the mycelia was 14%, the appearance was pale yellow, it can be observed from a microscope that the mycelia was deeply stained, thick and strong, and had obvious branches and segments.

### (4) Preparation and culture of fermentation tank

Formula of the medium (%): glucose 6, gluten powder 2.5, soybean meal 3.0, calcium carbonate 0.3, potassium dihydrogen phosphate 0.02, soybean oil 0.1, HABA 0.01, pH6.2, it was prepared with drinking water. The loading quantity was 700L in a 1000L fermentation tank, the medium was subjected to sterilization at the temperature of 121-125°C and the pressure of 0.10-0.13Mpa for 30 minutes, after cooling, the seed liquid was inoculated with the inoculation amount of 10%, the culture temperature was 29±2°C, the stirring rotation speed was 220 r/min, the ventilation ratio was 1:0.8 v/v/m. Soybean oil was added after 48 hours of culture, 0.3% was added once a day, in total, the amount of soybean oil supplemented during the whole fermentation process was 1.2%, the fermentation cycle was 168 hours, the concentration of the mycelia was about 40%, the appearance was dark yellow, pH 8.2, after the pretreatment of the fermentation liquid, the titer of stallimycin was determined to be 5360ug/mL by high performance liquid chromatography (HPLC).

### Example 3: Preparation of stallimycin

### (1) The strain and the preparation and culture of slant spore are seen in example 1

### (2) Preparation of shake flask mycelia

Formula of the medium (%): glucose 2, corn steep liquor 2.5, calcium carbonate 1.0, ammonium sulfate 0.1, potassium dihydrogen phosphate 0.01, pH6.0, it was prepared with drinking water, the loading quantity was 200mL in a 1000mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was about 12%, the appearance was pale yellow, it can be observed from a microscope that the mycelia was deeply stained and had branches and segments.

### (3) Preparation and culture of seed tank

Formula of the medium (%): glucose 2, corn steep liquor 2.5, calcium carbonate 1.0, ammonium sulfate 0.1, potassium dihydrogen phosphate 0.01, pH6.0, it was prepared with drinking water. The loading quantity was 65L in a 100L seed tank, the medium was subjected to sterilization at the temperature of 121-125°C and the pressure of 0.10-0.13Mpa for 30 minutes, after cooling, the mycelia liquid of the shake flask was inoculated, then it was cultivated at the temperature of 29±2°C, stirring rotation speed 220 r/min, ventilation ratio 1:1 v/v/m for 40 hours, the concentration of the mycelia was 13%, the appearance was pale yellow, it can be observed from a microscope that the mycelia was deeply stained, thick and strong, and had obvious branches and segments.

### (4) Preparation and culture of fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1, pH6.2, it was prepared with drinking water. The loading quantity was 700L in a 1000L fermentation tank , the medium was subjected to sterilization at the temperature of 121-125°C and the pressure of 0.10-0.13Mpa for 30 minutes, after cooling, the seed liquid was inoculated with the inoculation amount of 10%, the culture temperature was 29±2°C, the stirring speed was 220 r/min, the ventilation ratio was 1:0.8 v/v/m, soybean oil was added after 48 hours of culture, 0.5% was added once a day, in total, the amount of soybean oil supplemented during the whole fermentation process was 2%, the fermentation cycle was 168 hours, the concentration of the mycelia was about 38%,the appearance was dark yellow, pH 8.4, after the pretreatment of the fermentation liquid, the titer of stallimycin was determined to be 5400ug/mL by high performance liquid chromatography (HPLC).

### Example 4: Preparation of stallimycin

### (1) The strain and the preparation and culture of slant spore are seen in example 1

### (2) Preparation of shake flask mycelia

Formula of the medium (%): glucose 2, corn steep liquor2.5, calcium carbonate 1.0, ammonium sulfate 0.1, potassium dihydrogen phosphate 0.01, pH6.0, it was prepared with drinking water, the loading quantity was 200mL in a 1000mL triangular flask. The medium was subjected to sterilization at the temperature of 121-125°C and at the pressure of 0.10-0.13 MPa for 30 minutes. After cooling, the medium blocks comprising spores were dug out and inoculated into the seed culture medium, then at the temperature of 29±2°C, they were cultured on a shaker with a rotation speed of 220r/min for 40 hours, the concentration of mycelia was about 14%, the appearance was pale yellow, it can be observed from a microscope that the mycelia was deeply stained and had branches and segments.

### (3) Preparation and culture of seed tank

Formula of the medium (%): glucose 2, corn steep liquor 2.5, calcium carbonate 1.0, ammonium sulfate 0.1, potassium dihydrogen phosphate 0.01, pH6.0, it was prepared with drinking water. The loading quantity was 65L in a 100L seed tank , the medium was subjected to sterilization at the temperature of 121-125°C and the pressure of 0.10-0.13Mpa for 30 minutes, after cooling, the mycelia liquid of the shake flask was inoculated, then it was cultivated at the temperature of 29±2°C, stirring speed 220 r/min, ventilation ratio 1:1 v/v/m for 40 hours, the concentration of the mycelia was 14%, the appearance was pale yellow, it can be observed from a microscope that the mycelia was deeply stained, thick and strong, had obvious branches and segments.

### (4) Preparation and culture of fermentation tank

Formula of the medium (%): glucose 6, gluten powder 2.5, soybean meal 3.0, calcium carbonate 0.3, potassium dihydrogen phosphate 0.02, soybean oil 0.1, HABA 0.01, pH6.2, it was prepared with drinking water. The loading quantity was 700L in a 1000L fermentation tank, the medium was subjected to sterilization at the temperature of 121-125°C and the pressure of 0.10-0.13Mpa for 30 minutes, after cooling, the seed liquid was inoculated with the inoculation amount of 10%, the culture temperature was 29±2°C, the stirring speed was 220 r/min, the ventilation ratio was 1:0.8 v/v/m. Soybean oil was added after 48 hours of culture, 1.0% was added once 24 hours, in total, the amount of soybean oil supplemented during the whole fermentation process was 4%, the fermentation cycle was 168 hours, the concentration of the mycelia was about 36%, the appearance was dark yellow, pH 7.8, after the pretreatment of the fermentation liquid, the titer of stallimycin was determined to be 5210ug/mL by high performance liquid chromatography (HPLC).

### Example 5: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia was about 35%, the pH was 8.3, and the appearance was dark yellow. 3 fold (volume) of 80% ethanol was added to the fermentation liquid, soaked for 30 minutes, filtered, the filtrate was collected, and the titer of stallimycin was determined to be 5500ug/mL by high performance liquid chromatography (HPLC).

### Example 6: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.05, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia was about 35%, the pH was 8.0, and the appearance was dark yellow. 3 fold (volume) of 80% ethanol was added to the fermentation liquid, soaked for 30 minutes, filtered, the filtrate was collected, and the titer of stallimycin was determined to be 5220ug/mL by high performance liquid chromatography (HPLC).

### Example 7: Preparation of stallimycin

### (1) The strain and the preparation and culture of slant spore are seen in comparative example 1

### (2) Preparation and culture of shake flask seed culture medium are seen in comparative example 1

### (3) Preparation and culture of shake flask fermentation medium

Formula of the medium (%): glucose 6, gluten powder 2.5, soybean meal 3, sodium chloride 0.25, calcium carbonate 0.3,potassium dihydrogen phosphate 0.02, HABA 0.01, soybean oil 0.1. pH6.2, it was prepared with drinking water. The loading quantity was 50mL in a 500mL triangular flask , the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia was about 34%, the pH was 8.5, and the appearance was dark yellow. 3 fold (volume) of 80% ethanol was added to the fermentation liquid, soaked for 30 minutes, filtered, the filtrate was collected, and the titer of stallimycin was determined to be 1610 ug/mL by high performance liquid chromatography (HPLC).

### Example 8: Preparation of stallimycin

### (1) The strain and the preparation and culture of slant spore are seen in comparative example 2

### (2) Preparation and culture of shake flask seed culture medium are seen in comparative example 2

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia was about 35%, the pH was 8.4, and the appearance was dark yellow. 3 fold (volume) of 80% ethanol was added to the fermentation liquid, soaked for 30 minutes, filtered, the filtrate was collected, and the titer of stallimycin was determined to be 2270ug/mL by high performance liquid chromatography (HPLC).

### Example 9: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% rapeseed oil was added every 24 hours (in total, the amount of rapeseed oil added during the whole fermentation process was 2%), they were cultivated for 168 hours and put into a bottle. The concentration of the mycelia was about 35%, the pH was 8.3, and the appearance was dark yellow. 3 fold (volume) of 80% ethanol was added to the fermentation liquid, soaked for 30 minutes, filtered, the filtrate was collected, and the titer of stallimycin was determined to be 5400ug/mL by high performance liquid chromatography (HPLC).

### Example 10: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask , the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% peanut oil was added every 24 hours (in total, the amount of peanut oil added during the whole fermentation process was 2%), they were cultivated for 168 hours and put into a bottle. The concentration of the mycelia was about 38%, the pH was 8.3, and the appearance was dark yellow. 3 fold (volume) of 80% ethanol was added to the fermentation liquid, soaked for 30 minutes, filtered, the filtrate was collected, and the titer of stallimycin was determined to be 5200ug/mL by high performance liquid chromatography (HPLC).

### Example 11: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask , the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% olive oil was added every 24 hours (in total, the amount of olive oil added during the whole fermentation process was 2%), they were cultivated for 168 hours and put into a bottle. The concentration of the mycelia was about 42%, the pH was 8.2, and the appearance was dark yellow. 3 fold (volume) of 80% ethanol was added to the fermentation liquid, soaked for 30 minutes, filtered, the filtrate was collected, and the titer of stallimycin was determined to be 5230ug/mL by high performance liquid chromatography (HPLC).

### Example 12: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%):glucose 6, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask , the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% sunflower oil was added every 24 hours (in total, the amount of sunflower oil added during the whole fermentation process was 2%), they were cultivated for 168 hours and put into a bottle. The concentration of the mycelia of the fermentation liquid was about 40%, the pH was 8.5, and the appearance was dark yellow. 3 fold (volume) of 80% ethanol was added to the fermentation liquid, soaked for 30 minutes, filtered, the filtrate was collected, and the titer of stallimycin was determined to be 5210ug/mL by high performance liquid chromatography (HPLC).

### Example 13: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 4, dextrin 2, gluten powder 2.5, soybean meal 3, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia of the fermentation liquid was about 37%, the pH was 8.2, and the appearance was dark yellow. 3 fold (volume) of 80% ethanol was added to the fermentation liquid, soaked for 30 minutes, filtered, the filtrate was collected, and the titer of stallimycin was determined to be 5450ug/mL by high performance liquid chromatography (HPLC).

### Example 14: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): dextrin 6, gluten powder 3, soybean meal 2.5, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask, the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia of the fermentation liquid was about 38%, the pH was 8.2, and the appearance was dark yellow. 3 fold (volume) of 80% ethanol was added to the fermentation liquid, soaked for 30 minutes, filtered, the filtrate was collected, and the titer of stallimycin was determined to be 5330ug/mL by high performance liquid chromatography (HPLC).

### Example 15: Preparation of stallimycin

### (1) The strain and the preparation and culture of slant spore are seen in example 1

### (2) Preparation and culture of shake flask seed culture medium are seen in example 1

### (3) Preparation and culture of shake flask fermentation medium

Formula of the medium (%): glucose 6, gluten powder 1.5, soybean meal 4, sodium chloride 0.25, calcium carbonate 0.3, potassium dihydrogen phosphate 0.02, HABA 0.01, soybean oil 0.1. pH6.2, it was prepared with drinking water. The loading quantity was 50mL in a 500mL triangular flask , the medium was subjected to moist heat sterilization at the temperature of 121-125°C and the pressure of 0.10-0.13Mpa for 30 minutes, then the seed liquid was inoculated into the fermentation medium, the inoculation amount was 10%, the culture temperature was 29±2°C. After culturing on the shaker with the rotation speed of 220 r/min for 48 hours, 0.5% soybean oil was added every 24 hours (in total, the amount of soybean oil added during the whole fermentation process was 2%), they were cultivated for 168 hours and put into a bottle. The concentration of the mycelia of the fermentation liquid was about 32%, the pH was 8.4, and the appearance was dark yellow. 3 fold (volume) of 80% ethanol was added to the fermentation liquid, soaked for 30 minutes, filtered, the filtrate was collected, and the titer of stallimycin was determined to be 5390ug/mL by high performance liquid chromatography (HPLC).

## Claims

1. A method for preparing stallimycin which comprising the step of fermenting streptomyces that can produce stallimycin in a fermentation medium comprising an available carbon source, an available nitrogen source and 3-hydroxy-4-aminobutyric acid, and the step of adding vegetable oil into the fermentation medium during the fermentation.

2. The method according to claim 1, wherein the streptomyces is selected from *Streptomyces distallicus* NRRL NO.2886, *Streptomyces distallicus* D32 or *Streptomyces distallicus* DZ206.

3. The method according to any one of claims 1-2, wherein the weight concentration of the 3-hydroxy-4-aminobutyric acid in the fermentation medium is 0.005%-0.05%, preferably 0.01%.

4. The method according to any one of claims 1-3, wherein during the fermentation means at the time when the fermentation has been conducted for 48-120 hours.

5. The method according to any one of claims 1-4, wherein the interval between the additions of vegetable oil is once every 24 hours.

6. The method according to any one of claims 1-5, wherein the weight ratio of the amount of vegetable oil added for each time to the fermentation medium is 0.3-1.0%, preferably 0.5%; the weight ratio of the total amount of the added vegetable oil to the fermentation medium is 1-4%, preferably 2%.

7. The method according to any one of claims 1-6, wherein the vegetable oil is selected from rapeseed oil, sunflower oil, peanut oil, soybean oil, olive oil or the mixtures thereof, preferably, soybean oil.

8. The method according to any one of claims 1-7, wherein the weight concentration of the available carbon source in the fermentation medium is 4-10%, preferably 5-8%, more preferably 6%; the weight concentration of the available nitrogen source in the fermentation medium is 2-7%, preferably 3-6%, more preferably 5%.

9. The method according to any one of claims 1-8, wherein the available carbon source is selected from lactose, maltose, dextrin, cassava flour, corn starch, glucose, wheat starch, potato starch, glycerol, vegetable oil or the mixtures thereof, preferably glucose, dextrin, soybean oil or the mixtures thereof; the available nitrogen source is selected from soybean meal, yellow soybean cake meal, yeast powder, corn steep liquor, dried corn steep liquor powder, fish meal, peptone, peanut cake powder, cottonseed cake meal, bran, gluten powder, ammonium sulfate, ammonium phosphate, ammonium chloride, ammonium nitrate or the mixtures thereof, preferably soybean meal, gluten powder or the mixture thereof.

10. The method according to any one of claims 1-9, wherein the temperature of fermentation is 20-40°C, the time of fermentation is 144-192 hours, the pH of the fermentation medium is 6.0-9.0.
